# EUROPÄISCHE PATENTSCHRIFT

(11) **EP 1 719 499 B1**
(45) Veröffentlichungstag und Bekanntmachung des Hinweises auf die Patenterteilung: **06.07.2011**
(21) Anmeldenummer: 06113285.8
(22) Anmeldetag: 28.04.2006
(51) Int. Cl.: A61K 8/81, A61Q 5/06

(54) **Polymerkombination für Haargele**
Polymer mixture for hair gels
Combinaison de polymères pour gels capillaires

(30) Priorität: 02.05.2005 DE 102005020705
(43) Veröffentlichungstag der Anmeldung: 08.11.2006
(73) Patentinhaber: Beiersdorf AG, 20245 Hamburg (DE)
(72) Erfinder: Pilzner, Anke, 22459, Hamburg (DE); Detert, Marion, 22455, Hamburg (DE); Otto, Sebastian, 20253, Hamburg (DE)

(56) Entgegenhaltungen:
- WO-A-99/13841
- US-A1- 2002 098 212
- US-A1- 2003 012 758
- US-A1- 2003 223 950
- US-A1- 2004 247 556
- WANG M ET AL: "ACRYLATES/HYDROXYESTERS ACRYLATES COPOLYMER IN PERSONAL CARE APPLICATIONS: ACUDYNE DHR DURABLE HOLD RESIN" RESEARCH DISCLOSURE, MASON PUBLICATIONS, HAMPSHIRE, GB, Bd. 478, Nr. 6, Februar 2004 (2004-02), XP007133360 ISSN: 0374-4353
- ROHM AND HAAS COMPANY: "Uses of Ethylenecarboxamide / Acrylamidomethylpropanesulfonate / Methacrylates polymer in personal care applications: Acudyne SCP styling conditioning polymer" RESEARCH DISCLOSURE, MASON PUBLICATIONS, HAMPSHIRE, GB, Bd. 478, Nr. 2, Februar 2004 (2004-02), XP007133356 ISSN: 0374-4353
- ANONYMOUS: "LuvimerÂ Pro 55 - Acrylates Terpolymer for Cosmetic compositions" IP.COM JOURNAL, IP.COM INC., WEST HENRIETTA, NY, US, 14. Januar 2005 (2005-01-14), XP013022837 ISSN: 1533-0001
- ANDREA KEENAN OF THE ROHM AND HAAS COMPANY: "Hair styling formulations containing Acudyne 180 hair fixative polymer and Aculyn rheology modifiers" RESEARCH DISCLOSURE, MASON PUBLICATIONS, HAMPSHIRE, GB, Bd. 478, Nr. 8, Februar 2004 (2004-02), XP007133362 ISSN: 0374-4353

## Beschreibung

Die vorliegende Erfindung betrifft wässrige oder wässrig-alkoholische kosmetische Zubereitungen zur Frisurgestaltung des menschlichen Kopfhaares enthaltend

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem so genannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Natürliches Haar hängt meist schlaff und ohne Volumen vom Kopf herab. Ein Ziel der Haarpflege ist es daher, dem Haar Fülle und Frisur zu geben. Zur temporären Verformung des Haares und Gestaltung (engl. Styling) vielseitiger Frisuren verwendet man Haarfestiger, meist in Form von Schaumfestigern oder Haarsprays. Beide haarkosmetischen Mittel sind in Ihrer Zusammensetzung ähnlich unterscheiden sich aber in ihrer Aufgabe und Anwendung. Schaumfestiger (auch Schaumhaarfestiger genannt) werden in der Regel zur Frisurgestaltung im feuchten Haar verteilt, Haarsprays zur Fixierung auf die (trockene) fertig gestylte Frisur gesprüht. Neben den wegen ihrer leichten und angenehmen Anwendbarkeit zunehmend beliebten Schaumhaarfestigern und flüssigen Haarfestigern werden auch Haarfestigergele angeboten.

Die Rezepturen für derartige Produkte sind vielfältig. Die Bestandteile müssen jedoch in ethanolischem, wässrig-ethanolischem oder wässrigem Medium verträglich sein.

Üblicherweise bestehen diese Mittel zur Festigung der Haare (Haarfestiger) aus Lösungen von filmbildenden natürlichen oder synthetischen Polymeren oder Polymerkombinationen. Es handelt sich dabei um nichtionische, anionische, kationische oder amphotere Polymere. Diese hinterlassen auf dem Haar nach der Applikation einen transparenten farblosen Film. Es werden eine Reihe von Anforderungen an diese Filme gestellt: sie sollen klar, glänzend, feuchtigkeitsunempfindlich, festigend, lang anhaltend, flexibel, nicht klebend sein, den natürlichen Griff des Haares nicht beeinträchtigen und sich auch wieder leicht durch handelsübliche Haarwaschmittel auswaschen lassen. Neben den Filmbildnern enthalten Stylingzubereitungen Verdickungsmittel, die dem Produkt die gewünschte Konsistenz geben.

Anionische oder amphotere Filmbildner werden in Haarfestigern eingesetzt, um eine gute und lang anhaltende Festigung der Frisur zu erreichen. Ein entscheidender Nachteil dieser Rohstoffe sind jedoch ihre mangelhaften pflegenden Eigenschaften: das Haar lässt sich schlecht kämmen und fühlt sich stumpf, rau und wenig gepflegt an. Nach dem Trocknen sind die Filme dann relativ hart, spröde und unflexibel. Nachteilig für den Verbraucher sind die taktilen Eigenschaften des Filmes sowie die Bildung von Rückständen beim Kämmen (Filmplaken). Diese rieseln auf Schultern oder Kleidung und lassen den Anwender ungepflegt erscheinen.

Eine Verbesserung in Bezug auf Griff und Kämmbarkeit wird bei Styling-Formulierungen auf Basis anionischer und amphoter Polymere typischerweise durch die Kombination mit kationischen Polymeren, kationischen Tensiden oder Silikonen (beispielsweise cyclische Polydimethylsiloxane (Cyclomethicone), Silikontenside (Polyethermodifizierte Siloxane) vom Typ Dimethicone) erreicht. Bei derartigen Zubereitungen besteht jedoch immer die Gefahr einer Ausfällung der Polymere durch die Bildung von Polymersalzen aus anionischen und kationischen Polymeren. Außerdem führt der Einsatz dieser kationischen Polymere oder kationischen Tenside meistens zu einer deutliche Abnahme der Festigungsleistung und des Langzeithaltes der Frisur.

Es war daher die Aufgabe der vorliegenden Erfindung, die Mängel des Standes der Technik zu beseitigen und Zubereitungen (insbesondere Mittel zur Frisurgestaltung) zu entwickeln, die zu einer guten Kämmbarkeit des Haares führen. Das Haar sollte sich nach der Anwendung nicht mehr stumpf anfühlen, sondern einen glatten, gut gepflegten Eindruck hinterlassen. Die Bildung von Filmplaken sollte unterdrückt sein. Festigungsleistung und Langzeithalt sollten gegenüber dem Stand der Technik erhöht sein.

Überraschend gelöst werden die Aufgaben durch eine wässrige oder wässrig-alkoholische kosmetische Zubereitung zur Frisurgestaltung des menschlichen Kopfhaares enthaltend
a) ein anionisches Acrylatharz mit einer Molmasse zwischen 50 000 und 150 000 und einer Glasübergangstemperatur von 75 °C bis 105 °C in einer Konzentration von 0,1 bis 10 Gew.%,
b) 0,1 bis 10 Gew.-% anionische und/oder nichtionische polymere Verdicker
c) 0,1 bis 5 Gew-% Additive zur Verbesserung der Eigenschaften von Polymerfilmen auf dem Haar gewählt aus der Gruppe der Verbindungen der Silikonöle, quaternären Verbindungen und 2-Methyl-1,3-propandiol,
wobei sich die Gewichtsangaben auf das Gesamtgewicht der Zubereitung beziehen, dadurch gekennzeichnet, dass das anionisches Acrylatharz ein lineares Tetrapolymer mit zufälliger Verteilung der Monomere darstellt, wobei als Monomere Methacrylsäure, Hydroxyethylmethacrylat, Methylmethacrylat und Butylacrylat eingesetzt werden.

Die erfindungsgemäßen Zubereitungen führen bei der Anwendung auf dem Haar zu besonders weichen und flexiblen Filmen. Beim Griff ins Haar fühlt sich dieses deutlich weniger rau und stumpf an, als dies bei herkömmlichen Produkten der Fall ist. Zusätzlich verringert sich die Anzahl sichtbarer Filmplaken beim Auskämmen.

Insbesondere war es überraschend, dass die Verschlechterung des Langzeithaltes des Haares, welcher gewöhnlicherweise beim Zusatz von Pflegestoffen zu beobachten ist, deutlich reduziert ist.

Zwar kennt der Fachmann Wang M et al. "Acrylates/Hydroxyesters Acrylates copolymer in personal care applications: Acudyne DHR durable hold resign" research disclosure, Mason Publications, Hampshire, GB, Bd. 478, Nr. 6, Februar 2004 (2004-02), XP00713360 ISSN: 0374-4353,

Rohm and Haas Company: Uses of Ethylenecarboxamide/Acrylamidomethylpropansulfonate/Methacrtylates polymer in personal care applications: Acudyne SCP styling conditioning polymer" research disclosure, Mason Publications, Hampshire, GB, Bd. 478, Nr. 2, Februar 2004 (2004-02), XP00713356 ISSN: 0374-4353,

"Luvimer Pro 55 - Acrylates terpolymerfor cosmetic compositions" IP.Com Journal, IP. Com Inc., West Henrietta, NY, US, 14.Januar 2005 (2005-01-14), XP013022837 ISSN: 1533-0001,

Andrea Keenan of the Rohm and Haas Company: "Hair styling formulations containing Acudyne 180 hair fixative polymer and Aculyn rheology modifiers" research disclosure, Mason Publications, Hampshire, GB, Bd. 478, Nr. 8, Februar 2004 (2004-02), XP007133602 ISSN: 0374-4353,

sowie die US 2002/098212, WO 99/13841, US 2003/012758, US 20041247556 und US 2003/223950, doch konnten diese Schriften nicht den Weg zur vorliegenden Erfindung weisen.

Es ist erfindungsgemäß bevorzugt, wenn das anionische Acrylatharz mit einer Molmasse zwischen 50 000 und 150 000 und einer Glasübergangstemperatur von 75 °C bis 105 °C in einer Konzentration von 0,5 bis 5,0 Gew.-%, bezogen auf das Gesamtgewicht der Zubereitung, in dieser enthalten ist.

Erfindungsgemäß bevorzugt ist es außerdem, wenn die erfindungsgemäße Zubereitung 0,5 bis 7,0 Gew.-% anionische und/oder nichtionische polymere Verdicker, bezogen auf das Gesamtgewicht der Zubereitung enthält.

Nicht zuletzt ist es erfindungsgemäß bevorzugt, wenn die erfindungsgemäße Zubereitung 0,5 bis 2,5 Gew-% Additive zur Verbesserung der Eigenschaften von Polymerfilmen auf dem Haar enthält, wobei sich die Gewichtsangaben auf das Gesamtgewicht der Zubereitung beziehen.

Es ist erfindungsgemäß, wenn das anionisches Acrylatharz die folgende Struktur aufweist: 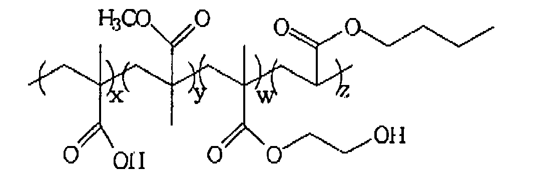 das heißt, wenn es sich um ein lineares Tetrapolymer mit zufälliger Verteilung der Monomere handelt, wobei als Monomere Methacrylsäure, Hydroxyethylmethacrylat, Methylmethacrylat und Butylacrylat eingesetzt werden.

Erfindungsgemäß vorteilhafte anionische polymere Verdicker (Gelbildner) sind die Polyacrylate. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alcylacrylat-Copolymere durch die folgende Struktur aus:

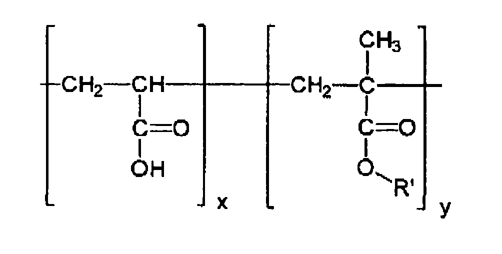

Darin stellen R' einen langkettigen Alkylrest und x und y Zahlen dar, welche den jeweiligen stöchiometrischen Anteil der jeweiligen Comonomere symbolisieren.

Erfindungsgemäß bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Ferner vorteilhaft sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Carbopol 1382, Carbopol ETD 2020, Carbopol Ultrez 21, Pemulen TR1 und Pemulen TR2 bei der NOVEON Inc. erhältlichen.

Vorteilhaft sind ferner Verbindungen, die die INCI-Bezeichnung "acrylates/C12-24 pareth-25 acrylate copolymer" (unter der Handelsbezeichnungen Synthalen® W2000 bei der 3V Inc. erhältlich), die die INCI-Bezeichnung "acrylates/steareth-20 methacrylate copolymer" (unter der Handelsbezeichnungen Aculyn® 22 bei der International Specialty Products Corp. erhältlich), die die INCI-Bezeichnung "acrylates/beheneth-25 methacrylate copolymer" (unter der Handelsbezeichnung Aculyn® 28 bei der International Specialty Products Corp. erhältlich), die die INCI-Bezeichnung "acrylates/steareth-20 itaconate copolymer" (unter der Handelsbezeichnungen Structure 2001® bei der National Starch erhältlich), die die INCI-Bezeichnung acrylates/aminoacrylates/C10-30 alkyl PEG-20 itaconate copolymer" (unter der Handelsbezeichnungen Structure Plus® bei der National Starch erhältlich) und ähnliche Polymere.

Erfindungsgemäß bevorzugte Ausführungsformen der vorliegenden Erfindung sind dadurch gekennzeichnet, dass als Polyacrylatverdicker Acrylat-Alkylacrylat-Copolymere eingesetzt werden.

Die erfindungsgemäß besonders bevorzugten Hydrokolloide sind: Carbomer (Carbopol 981 oder Carbopol 980) und Acrylates/C 10-30 Alkyl Acrylate Crosspolymer (Carbopol ETD 2020 oder Carbopol Ultrez 21).

Als nichtionische, verdickende Polymere, welche gewählt werden aus der Gruppe der Verbindungen der Cellulosen, der Cellulosederivate und Xanthan Gummis, werden erfindungsgemäß bevorzugt Hydroxyethylcellulose (CAS 9004-62-0, z.B. Natrosol), Hydroxypropylcellulose (CAS 9004-64-2, z.B. Klucel HF), Hydroxymethylpropylcellulose (CAS 9004-65-3, z.B. Methocel) und Xanthan Gummis (INCI Xanthan Gum, z.B. Keltrol F) eingesetzt.

Es ist erfindungsgemäß vorteilhaft, wenn die Konzentration an beschriebenen anionischen Polyacrylatverdicker und der nichtionischen Verdicker (Cellulose, Cellulosederivate und Xanthan Gum) in der gelförmigen, wässrigen Phase von 0,01 bis 10 Gewichts-%, bevorzugt von 0,5 bis 7,5 Gewichts-% und besonders bevorzugt von 0,1 bis 5,0 Gewichts-%, jeweils bezogen auf das Gesamtgewicht der gelförmigen, wässrigen Phase, beträgt.

Erfindungsgemäß ist es, wenn ein oder mehrere Additive zur Verbesserung der Eigenschaften von Polymerfilmen auf dem Haar gewählt werden aus der Gruppe der Verbindungen der Silikonöle, quaternären Verbindungen und 2-Methyl-1,3-propandiol.

Bevorzugt im Sinne der vorliegenden Erfindung ist es, wen ein oder mehrere Additive zur Verbesserung der Eigenschaften von Polymerfilmen auf dem Haar gewählt werden aus der Gruppe der Verbindungen PEG/PPG-20/6 Dimethicon, Citrimoniumchlorid und 2-Methyl-1,3-propandiol.

Erfindungsgemäß besonders bevorzugt ist es, wenn als Additiv zur Verbesserung der Eigenschaften von Polymerfilmen auf dem Haar 2-Methyl-1,3-propandiol eingesetzt wird. Dieses kann beispielsweise unter dem Handelsnamen MP Diol bei der Firma Aldrich erworben werden.

Vorteilhafte Ausführungsformen der vorliegenden Erfindung sind dadurch gekennzeichnet, dass die Zubereitung 0,1 bis 30 Gew.-% Ethanol und bevorzugt 10 bis 20 Gew.-% Ethanol, jeweils bezogen auf das Gesamtgewicht der Zubereitung, enthält.

Auch ist es erfindungsgemäß vorteilhaft, wenn die Zubereitung 0,01 bis 0,5 Gew.-% Benzophenon-4 und bevorzugt 0,01 bis 0,1 Gew-% Benzophenon-4, jeweils bezogen auf das Gesamtgewicht der Zubereitung, enthält.

Es ist erfindungsgemäß von Vorteil, wenn die Zubereitung 0,01 bis 0,2 Gew.-% Parabene und bevorzugt 0,01 bis 0,05 Gew.-% Parabene, jeweils bezogen auf das Gesamtgewicht der Zubereitung, enthält.

Vorteilhaft im Sinne der vorliegenden Erfindung ist es, wenn die Zubereitung 0,01 bis 2,0 Gew.-% PEG-40 hydriertes Rizinusöl und bevorzugt 0,01 bis 0,75 Gew.-%, jeweils bezogen auf das Gesamtgewicht der Zubereitung, enthält.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Antioxidanien, Bakterizide, Parfüme, Emulgatoren, weichmachende, anfeuchtende und/oder feuchthaltende Substanzen, Fette, Öle, Wachse, Farbstoffe, Pigmente, die eine färbende Wirkung haben, Verdickungsmittel oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Auch die Einarbeitung von UV-Lichtschutzfiltern ist erfindungsgemäß vorteilhaft. Die aufgeführte Liste an Zusatzstoffen soll selbstverständlich nicht limitierend sein.

Es ist erfindungsgemäß bevorzugt, wenn die erfindungsgemäße Zubereitung in Form eines Gels vorliegt, welches dadurch gekennzeichnet ist, dass die gelförmige, wässrige Phase eine Fließgrenze von 0,5 Pa bis 60 Pa aufweist.

Die erfindungsgemäße Zubereitung wird erfindungsgemäß vorteilhaft als Haargel verwendet.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

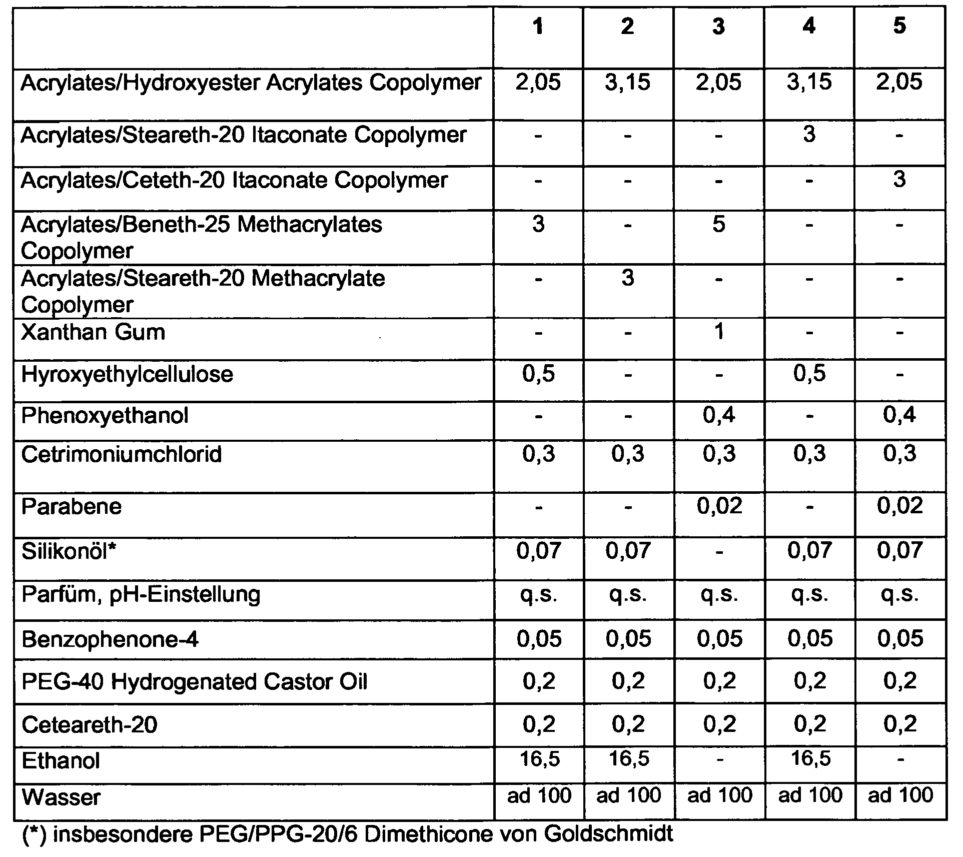

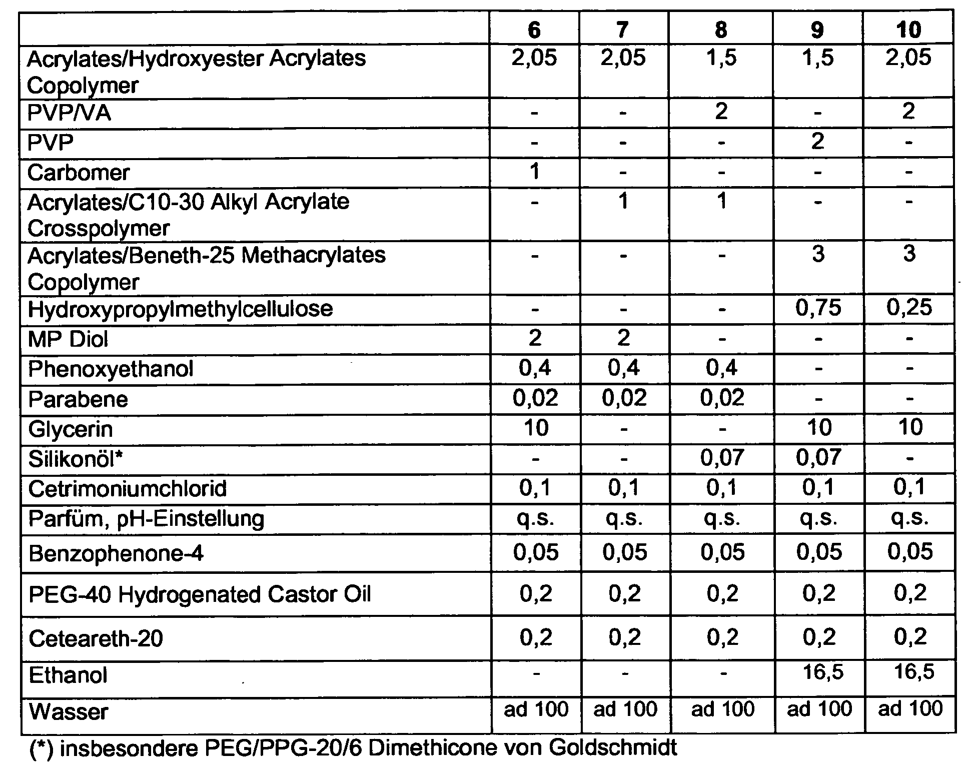

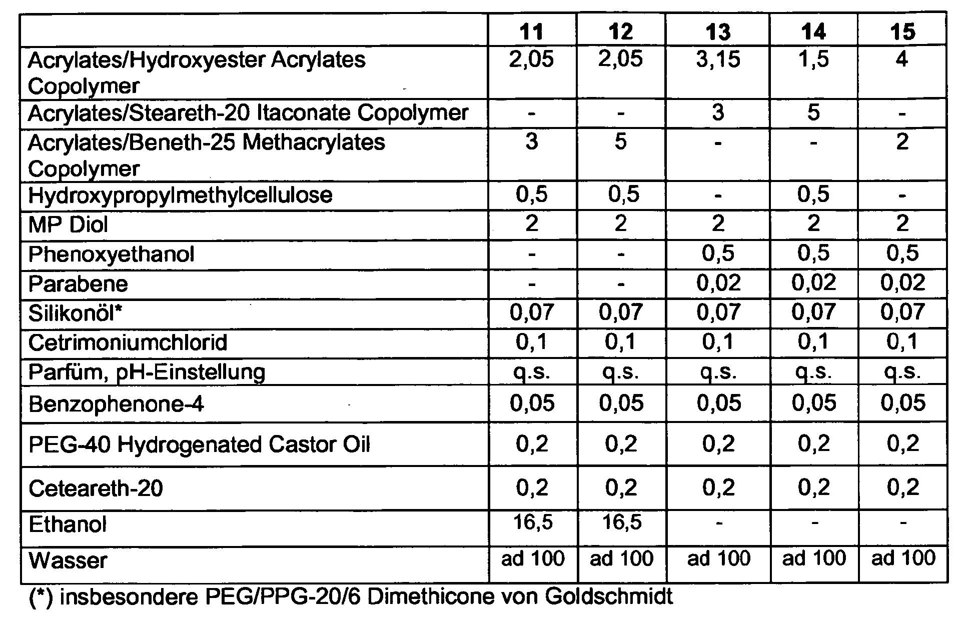

## Patentansprüche

1. Wässrige oder wässrig-alkoholische kosmetische Zubereitung zur Frisurgestaltung des menschlichen Kopfhaares enthaltend
a) ein anionisches Acrylatharz mit einer Molmasse zwischen 50 000 und 150 000 und einer Glasübergangstemperatur von 75 °C bis 105 °C in einer Konzentration von 0,1 bis 10 Gew.-%,
b) 0,1 bis 10 Gew.-% anionische und/oder nichtionische polymere Verdicker
c) 0,1 bis 5 Gew-% Additive zur Verbesserung der Eigenschaften von Polymerfilmen auf dem Haar gewählt aus der Gruppe der Verbindungen der Silikonöle, quaternären Verbindungen und 2-Methyl-1,3-propandiol,
wobei sich die Gewichtsangaben auf das Gesamtgewicht der Zubereitung beziehen, **dadurch gekennzeichnet, dass** das anionisches Acrylatharz ein lineares Tetrapolymer mit zufälliger Verteilung der Monomere darstellt, wobei als Monomere Methacrylsäure, Hydroxyethylmethacrylat, Methylmethacrylat und Butylacrylat eingesetzt werden.

2. Zubereitung nach Anspruch 1, **dadurch gekennzeichnet, dass** als anionische und /oder nichtionische polymere Verdicker gewählt werden aus der Gruppe der Verbindungen Polyacrylat-, Cellulose-, Cellulosederivatverdicker und/oder Xanthan Gum.

3. Zubereitung nach einem der vorhergehenden Ansprüche, **dadurch gekennzeichnet,**
**dass** als Polyacrylatverdicker Acrylat-Alkylacrylat-Copolymere eingesetzt werden.

4. Zubereitung nach einem der vorhergehenden Ansprüche, **dadurch gekennzeichnet,**
**dass** ein oder mehrere Additive zur Verbesserung der Eigenschaften von Polymerfilmen auf dem Haar gewählt werden aus der Gruppe der Verbindungen PEG/PPG-20/6 Dimethicon, Citrimoniumchlorid und 2-Methyl-1,3-propandiol.

5. Zubereitung nach einem der vorhergehenden Ansprüche, **dadurch gekennzeichnet,**
**dass** als Additiv zur Verbesserung der Eigenschaften von Polymerfilmen auf dem Haar 2-Methyl-1,3-propandiol eingesetzt wird.

6. Zubereitung nach einem der vorhergehenden Ansprüche, **dadurch gekennzeichnet,**
**dass** die Zubereitung 0,1 bis 20 Gew.-% Ethanol, bezogen auf das Gesamtgewicht der Zubereitung, enthält.

7. Zubereitung nach einem der vorhergehenden Ansprüche, **dadurch gekennzeichnet,**
**dass** die Zubereitung 0,01 bis 0,5 Gew.-% Benzophenon-4, bezogen auf das Gesamtgewicht der Zubereitung, enthält.

8. Zubereitung nach einem der vorhergehenden Ansprüche, **dadurch gekennzeichnet,**
**dass** die Zubereitung 0,01 bis 2,0 Gew.-% PEG-40 hydriertes Rizinusöl, bezogen auf das Gesamtgewicht der Zubereitung, enthält.

## Claims

1. Aqueous or aqueous-alcoholic cosmetic preparation for styling human head hair, comprising
a) an anionic acrylate resin with a molar mass between 50 000 and 150 000 and a glass transition temperature from 75°C to 105°C in a concentration of from 0.1 to 10% by weight,
b) 0.1 to 10% by weight of anionic and/or nonionic polymeric thickeners,
c) 0.1 to 5% by weight of additives for improving the properties of polymer films on the hair, selected from the group of the compounds of the silicone oils, quaternary compounds and 2-methyl-1,3-propanediol,
where the weight data refer to the total weight of the preparation, **characterized in that** the anionic acrylate resin is a linear tetrapolymer with random distribution of the monomers, where methacrylic acid, hydroxyethyl methacrylate, methyl methacrylate and butyl acrylate are used as monomers.

2. Preparation according to Claim 1, **characterized in that** anionic and/or nonionic polymeric thickeners are selected from the group of the compounds polyacrylate, cellulose, cellulose derivative thickeners and/or xanthan gum.

3. Preparation according to one of the preceding claims, **characterized in that** acrylate-alkyl acrylate copolymers are used as polyacrylate thickeners.

4. Preparation according to one of the preceding claims, **characterized in that** one or more additives for improving the properties of polymer films on the hair are selected from the group of the compounds PEG/PPG-20/6 dimethicone, cetrimonium chloride and 2-methyl-1,3-propanediol.

5. Preparation according to one of the preceding claims, **characterized in that** 2-methyl-1,3-propanediol is used as additive for improving the properties of polymer films on the hair.

6. Preparation according to one of the preceding claims, **characterized in that** the preparation comprises 0.1 to 20% by weight of ethanol, based on the total weight of the preparation.

7. Preparation according to one of the preceding claims, **characterized in that** the preparation comprises 0.01 to 0.5% by weight of benzophenone-4, based on the total weight of the preparation.

8. Preparation according to one of the preceding claims, **characterized in that** the preparation comprises 0.01 to 2.0% by weight of PEG-40 hydrogenated castor oil, based on the total weight of the preparation.

## Revendications

1. Préparation cosmétique aqueuse ou aqueuse-alcoolique pour le coiffage des cheveux humains, contenant
a) une résine anionique d'acrylate présentant une masse molaire entre 50 000 et 150 000 et une température de transition vitreuse de 75°C à 105°C en une concentration de 0,1 à 10% en poids,
b) 0,1 à 10% en poids d'épaississants polymères anioniques et/ou non ioniques
c) 0,1 à 5% en poids d'additifs pour améliorer les propriétés de films polymères sur les cheveux, choisis dans le groupe formé par les composés des huiles de silicone, les composés quaternaires et le 2-méthyl-1,3-propanediol,
où les indications en poids se rapportent au poids total de la préparation, **caractérisée en ce que** la résine anionique d'acrylate représente un tétrapolymère linéaire avec une répartition aléatoire des monomères, l'acide méthacrylique, le méthacrylate d'hydroxyéthyle, le méthacrylate de méthyle et l'acrylate de butyle étant utilisés comme monomères.

2. Préparation selon la revendication 1, **caractérisée en ce qu'**on choisit les épaississants polymères anioniques et/ou non ioniques dans le groupe formé par les composés épaississants à base de polyacrylate, de cellulose, de dérivés de cellulose et/ou la gomme de xanthane.

3. Préparation selon l'une quelconque des revendications précédentes, **caractérisée en ce qu'**on utilise, comme épaississants à base de polyacrylate, des copolymères d'acrylate-acrylate d'alkyle.

4. Préparation selon l'une quelconque des revendications précédentes, **caractérisée en ce qu'**un ou plusieurs additifs pour améliorer les propriétés de films polymères sur les cheveux sont choisis dans le groupe formé par les composés PEG/PPG-20/6 Dimethicon, chlorure de citrimonium et 2-méthyl-1,3-propanediol.

5. Préparation selon l'une quelconque des revendications précédentes, **caractérisée en ce qu'**on utilise, comme additif pour améliorer les propriétés de films polymères sur les cheveux, le 2-méthyl-1,3-propanediol.

6. Préparation selon l'une quelconque des revendications précédentes, **caractérisée en ce que** la préparation contient 0,1 à 20% en poids d'éthanol, par rapport au poids total de la préparation.

7. Préparation selon l'une quelconque des revendications précédentes, **caractérisée en ce que** la préparation contient 0,01 à 0,5% en poids de benzophénone-4, par rapport au poids total de la préparation.

8. Préparation selon l'une quelconque des revendications précédentes, **caractérisée en ce que** la préparation contient 0,01 à 2,0% en poids d'huile de ricin hydrogénée par du PEG-40, par rapport au poids total de la préparation.
